(19) Europäisches Patentamt
European Patent Office
Office européen des brevets

(11) Veröffentlichungsnummer: **0 011 134**
**B1**

(12) # EUROPÄISCHE PATENTSCHRIFT

(45) Veröffentlichungstag der Patentschrift: **05.05.82**

(51) Int. Cl.³: **C 07 J 21/00**, A 61 K 31/58

(21) Anmeldenummer: **79103870.6**

(22) Anmeldetag: **09.10.79**

(54) **Hydrocortison-orthoester, diese enthaltende pharmazeutische Zubereitungen und Verfahren zu ihrer Herstellung.**

(30) Priorität: **09.11.78 DE 2848584**

(43) Veröffentlichungstag der Anmeldung: **28.05.80 Patentblatt 80/11**

(45) Bekanntmachung des Hinweises auf die Patenterteilung: **05.05.82 Patentblatt 82/18**

(84) Benannte Vertragsstaaten: **AT BE CH DE FR GB IT NL SE**

(56) Entgegenhaltungen:
**DE-A-2 122 351**
**US-A-3 147 249**

(73) Patentinhaber: **Merck Patent Gesellschaft mit beschränkter Haftung, Frankfurter Strasse 250, D-6100 Darmstadt (DE)**

(72) Erfinder: **Baumgarth, Manfred, Dr. Chem., Sachsenstrasse 53, D-6100 Darmstadt (DE)**
Erfinder: **Orth, Dieter, Dr. Chem., Jahnstrasse 83, D-6100 Darmstadt (DE)**
Erfinder: **Harting, Jürgen, Dr., Heidelbergerstrasse 123, D-6100 Darmstadt (DE)**
Erfinder: **Schaefer, Hans, Prof. Dr. Chem., Avenue des Pins Domaine du Pimeau, F-06600 Antibes (FR)**
Erfinder: **Zesch, Achim, Prof. Dr., Graneweg 11, D-1000 Berlin 28 (DE)**

EP 0 011 134 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG.

# Hydrocortison-orthoester, diese enthaltende pharmazeutische Zubereitungen und Verfahren zu ihrer Herstellung

Die Erfindung betrifft neue Hydrocortison-orthoester der allgemeinen Formel I

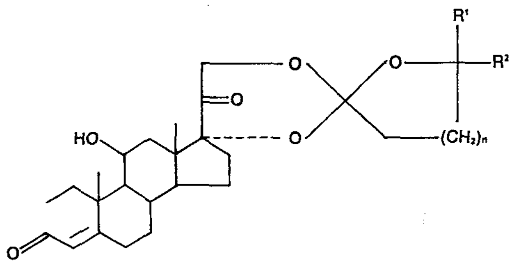

I

worin $R^1$ und $R^2$ jeweils H oder $CH_3$ und
n 1 oder 2
bedeuten.

Der Erfindung lag die Aufgabe zugrunde, neue Verbindungen mit wertvollen Eigenschaften aufzufinden, insbesondere solche, die zur Herstellung von Arzneimitteln verwendet werden können. Diese Aufgabe wurde durch die Bereitstellung der Verbindungen der Formel I gelöst.

Es wurde gefunden, dass die Verbindungen der Formel I bei guter Verträglichkeit wertvolle pharmakologische Eigenschaften besitzen. Insbesondere treten antiphlogistische Wirkungen auf, die sich z.B. auf eine antiproliferative Wirkungskomponente [feststellbar z.B. analog der Methodik von Rudas, Drug Research 10, 226 (1969)], eine antiexsudative Wirkungskomponente [feststellbar z.B. analog der von Hotovy und Kapff, Arch. Int. Pharmacodyn. 111, 420-436 (1957) beschriebenen Methode; Granulombeuteltest] eine thymolytische Wirkungskomponente [feststellbar z.B. analog der Methodik von Steelman et al., Steroids 1, 163 (1963)], und eine ACTH-beeinflussende Wirkungskomponente [feststellbar aufgrund der Hemmung einer Nebennierenhypertrophie analog der Methodik von Bohus, B., Acta Physiol. Acad. Sci. Hung. 29, 203 (1966)] zurückführen lassen. Daher eignen sich die Verbindungen der Formel I z.B. zur Bekämpfung hartnäckiger Allergien und anderer entzündlicher Erkrankungen der Haut, ferner zur Behandlung rheumatischer Arthritis. Diese Wirkungen sind nach den dafür üblichen Untersuchungsmethoden erfassbar.

Die Verbindungen der Formel I können daher als Arzneimittelwirkstoffe in der Human- und Veterinärmedizin und auch als Zwischenprodukte zur Herstellung anderer Arzneimittelwirkstoffe verwendet werden.

Ähnliche Steroid-orthoester sind aus der DE-A-2 122 351 und insbesondere aus der US-A-3-147 249 bekannt. Die vorliegenden neuen Hydrocortison-orthoester sind jedoch den bekannten Verbindungen hinsichtlich ihrer antiphlogistischen Wirkung überlegen.

Gegenstand der Erfindung sind die Verbindungen der Formel I. In der Formel I bedeuten die Reste $R^1$ und $R^2$ vorzugsweise H; der Parameter n hat vorzugsweise den Wert 2. Dementsprechend sind Gegenstand der Erfindung insbesondere diejenigen Verbindungen der Formel I, in denen mindestens einer der Reste $R^1$ und $R^2$ bzw. der Parameter n eine der vorstehend angegebenen bevorzugten Bedeutungen hat.

Gegenstand der Erfindung ist ferner ein Verfahren zur Herstellung der Verbindungen der Formel I, dadurch gekennzeichnet, dass man Hydrocortison mit einem Lacton der allgemeinen Formel II

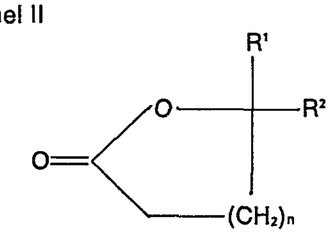

II

worin
$R^1$, $R^2$ und n die oben angegebene Bedeutung haben,
oder einem seiner reaktionsfähigen Derivate umsetzt.

Die Hydrocortison-orthoester der allgemeinen Formel I werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; ferner z.B. DE-OS 2 122 351) beschrieben sind, und zwar unter Reaktionsbedingungen, wie sie für diese Umsetzungen bekannt und geeignet sind. Dabei kann man auch von an sich bekannten, hier nicht näher erwähnten Varianten Gebrauch machen.

Als reaktionsfähige Derivate der Lactone der Formel II kommen bevorzugt die entsprechenden Lactonacetale der allgemeinen Formel III in Betracht,

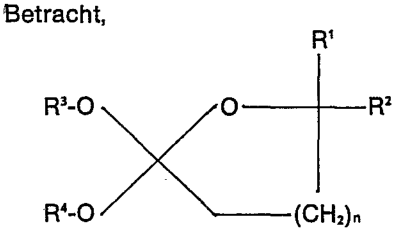

III

worin
$R^3$ und $R^4$ jeweils Alkyl mit 1-4 C-Atomen oder zusammen $-C_mH_{2m}-$ und
m 2, 3 oder 4 bedeuten und
$R^1$, $R^2$ und n die angegebene Bedeutung haben,
ferner auch die Lactoniumsalze der allgemeinen Formel IV

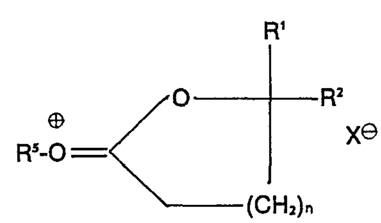

IV

worin

$R^5$ Alkyl mit 1-4 C-Atomen und
$X^{\ominus}$ ein Anion wie $BF_4^{\ominus}$ oder
$SbF_6^{\ominus}$ bedeuten und
$R^1$, $R^2$ und n die angegebene Bedeutung haben.

In den Lactonderivaten der Formeln III und IV bedeuten $R^3$, $R^4$ und $R^5$ vorzugsweise Methyl oder Äthyl, $R^3$ und $R^4$ zusammen auch vorzugsweise $-CH_2CH_2-$ oder $-CH_2CH_2CH_2-$, aber auch Propyl, Isopropyl, Butyl, Isobutyl oder sek.-Butyl, $R^3$ und $R_4$ zusammen auch $-CH_2CH_2CH_2CH_2-$, $-CH_2-CH(CH_3)-$, $-CH(CH_3)-CH(CH_3)-$, $-CH_2-CH(C_2H_5)-$ usw.

Die Ausgangsstoffe der Formeln III und IV sind teilweise bekannt [vgl. z.B. Chem. Ber. 89, 2060-2079 (1956); J. Org. Chem. 42, 3207 (1977)]. Die nicht bekannten Verbindungen der Formeln III und IV können in Analogie zu den bekannten nach an sich bekannten Verfahren hergestellt werden. Die Ausgangsstoffe können gewünschtenfalls auch in situ hergestellt werden, derart, dass man sie aus dem Reaktionsgemisch nicht isoliert, sondern sofort weiter zu Verbindungen der Formel I umsetzt.

Die Herstellung der Orthoester der Formel I gelingt durch Umsetzung von Hydrocortison mit Lactonen der Formel II, vorzugsweise jedoch mit Lactonacetalen der Formel III.

Die Reaktion von Hydrocortison mit den Lactonacetalen III wird zweckmässig in einem inerten organischen Lösungsmittel vorgenommen, bevorzugt in Amiden wie Dimethylformamid, Dimethylacetamid oder Formamid, Sulfoxiden wie Dimethylsulfoxid, Nitrilen wie Acetonitril, bei Temperaturen zwischen 0 und 150, vorzugsweise 100 und 120°, in Gegenwart eines sauren Katalysators, z.B. einer Mineralsäure wie Salzsäure, Schwefelsäure, einer Sulfonsäure wie p-Toluolsulfonsäure oder einer Lewis-Säure wie $BF_3$ oder $AlCl_3$. Als Lösungsmittel eignet sich auch ein Überschuss des Lactonacetals III.

Die Reaktion von Hydrocortison mit den Lactoniumsalzen IV erfolgt zweckmässig ebenfalls in Gegenwart eines inerten organischen Lösungsmittels bzw. Suspensionsmittels, z.B. eines halogenierten Kohlenwasserstoffs wie Chloroform, Methylenchlorid, Tetrachlorkohlenstoff, 1,2- oder 1,1-Dichloräthan, eines Kohlenwasserstoffs wie Benzol oder Toluol oder eines Äthers wie Diäthyläther oder Dioxan in Gegenwart einer Base wie Ammoniak, Triäthylamin oder Pyridin bei Temperaturen zwischen etwa 0 und etwa 50°.

Gegenstand der Erfindung ist ferner die Verwendung der neuen Verbindungen der Formel I zur Herstellung pharmazeutischer Zubereitungen, insbesondere auf nicht-chemischem Wege. Die Verbindungen I können zusammen mit mindestens einem festen, flüssigen und/oder halbflüssigen Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I.

Diese Zubereitungen können als Arzneimittel in der Human und Veterinärmedizin verwendet werden. Als Trägerstoffe kommen organische oder anorganische Substanzen in Frage, die sich insbesondere für die topische Applikation eignen und mit den neuen Verbindungen nicht reagieren, beispielsweise Wasser, pflanzliche Öle, Kohlenwasserstoffe wie alkylierte Naphthaline, halogenierte Kohlenwasserstoffe wie $CF_2Cl_2$ (z.B. für Aerosole), Benzylalkohole, Polyäthylenglycole, Glycerintriacetat, Gelatine, Kohlenhydrate wie Lactose oder Stärke, Magnesiumstearat, Talk, Vaseline®. Zur topischen Anwendung dienen insbesondere Lösungen, Lotionen, Emulsionen, Sprays (Aerosole), Salben, Cremes, Pasten oder Puder. Die neuen Verbindungen können auch lyophilisiert werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z.B. ein oder mehrere Antibiotika wie Gentamycin und/oder Antimykotika und/oder andere topisch wirkende Substanzen.

Die neuen Verbindungen werden in der Regel in Analogie zu bekannten, im Handel befindlichen Entzündungshemmern (z.B. Hydrocortison-17-butyrat) verabreicht. Bei der topischen Applikation in Kombination mit dafür geeigneten Trägerstoffen kann eine gute Aktivität über grössere Verdünnungsbereiche festgestellt werden. Beispielsweise zeigen sich Konzentrationen des Wirkstoffs zwischen etwa 0,05 und 1 Gewichtsprozent, bezogen auf das Gewicht des verwendeten Präparats, als wirksam zur Heilung von Entzündungen. Bevorzugt sind Konzentrationen von etwa 0,1 bis 0,5 Gewichtsprozent.

Jede der in den folgenden Beispielen genannten Verbindungen der Formel I ist zur Herstellung von pharmazeutischen Zubereitungen besonders geeignet. Vor- und nachstehend sind die Temperaturen in °C angegeben.

Beispiel 1

Ein Gemisch von 36,25 g Hydrocortison, 36 ml 2,2-Diäthoxytetrahydrofuran, 36 ml trockenem Dimethylformamid und 150 mg p-Toluolsulfonsäure wird 3 Stunden bei 115° gerührt, dann abgekühlt, mit 1,2 ml Pyridin versetzt und in Wasser gegossen. Man extrahiert mit Dichlormethan, wäscht den Extrakt mit Wasser, trocknet über Natriumsulfat und dampft ein. Nach chromatographischer Reinigung (Kieselgel; Dichlormethan/Petroläther/Aceton 5:5:2 + 0,1% Triäthylamin) erhält man 17α,21-Tetrahydrofuranyliden-2',2'-dioxy-4-pregnen-11β-ol-3,20-dion, F. 223 bis 225° (aus Aceton); $[\alpha]_D^{20}$ +120,4° (Chloroform).

Beispiele 2 bis 6

Analog Beispiel 1 erhält man aus Hydrocortison mit 2,2-Diäthoxy-5-methyl-tetrahydrofuran (Kp. 71-72°/14 mm), 2,2-Diäthoxy-5,5-dimethyl-tetrahydrofuran (Kp. 72-75°/14 mm), 2,2-Diäthoxy--tetrahydropyran, 6-Methyl-2,2-diäthoxy-tetrahydropyran oder 6,6-Dimethyl-2,2-diäthoxy-tetrahydropyran:

2. 5'-Methyl-17α,21-tetrahydrofuranyliden-2',2'--dioxy-4-pregnen-11β-ol-3,20-dion, F. 190 - 192°.
3. 5',5'-Dimethyl-17α,21-tetrahydrofuranyliden--2',2'-dioxy-4-pregnen-11β-ol-3,20-dion, F. 143 - 145° bzw. 185 - 186° (zwei Kristall-Modifikationen); $[\alpha]_D^{20}$ +105° (Chloroform).
4. 17α,21-Tetrahydropyranyliden-2',2'-dioxy-4--pregnen-11β-ol-3,20-dion, F. 234 - 236°; $[\alpha]_D^{20}$ +104,4° (Chloroform).
5. 6'-Methyl-17α,21-tetrahydropyranyliden-2',2'--dioxy-4-pregnen-11β-ol-3,20-dion.
6. 6',6'-Dimethyl-17α,21-tetrahydropyranyliden--2',2'-dioxy-4-pregnen-11β-ol-3,20-dion.

Beispiel 7

Analog Beispiel 1 erhält man aus Hydrocortison mit 2,2-Äthylendioxy-tetrahydropyran (Kp. 73 bis 76°/12 mm) bzw. mit 2,2-(Propylen-1,3-dioxy)--tetrahydropyran (Kp. 87 - 89°/14 mm) das in Beispiel 4 beschriebene Produkt.

Beispiel 8

Man dispergiert 1 g Triäthyloxonium-tetrafluorborat in 5 ml trockenem Methylenchlorid unter $N_2$, fügt 0,45 ml Butyrolacton hinzu und lässt über Nacht bei 25° stehen. Anschliessend tropft man 1,6 ml der so erhaltenen O-Äthylbutyrolactonium-tetrafluorboratlösung bei 25° unter Rühren zu einer Dispersion von 362 mg Hydrocortison in einer Lösung von 200 mg Triäthylamin in 18 ml trockenem Methylenchlorid innerhalb 15 Minuten. Das erhaltene Gemisch wird über $Al_2O_3$ filtriert. Aus dem Filtrat erhält man 17α,21-Tetrahydrofuranyliden-2',2'-dioxy-4-pregnen-11β-ol-3,20-dion, F. 223 - 225°.

Die folgenden Beispiele betreffen pharmazeutische Zubereitungen, die Verbindungen der Formel I enthalten (Prozentangaben sind Gewichtsprozente).

Beispiel A: Salbe

| | |
|---|---|
| 17α,21-Tetrahydropyranyliden-2',2'-dioxy--4-pregnen-11β-ol-3,20-dion | 0,25 % |
| Wollfett wasserfrei | 2,0 % |
| Paraffin dickflüssig | 10,0 % |
| Vaseline® weiss | ad 100,0 % |

Beispiel B: Creme

| | |
|---|---|
| 17α,21-Tetrahydropyranyliden-2',2'-dioxy--4-pregnen-11β-ol-3,20-dion | 0,5 % |
| Cetylalkohol | 9,0 % |
| Paraffin dickflüssig | 3,0 % |
| Glycerinmonostearat | 2,0 % |
| Propylenglykolmonostearat | 2,0 % |
| Glycerin | 2,0 % |
| feinstteilige Kieselsäure | 0,1 % |
| Vaseline® | 10,0 % |
| Polyoxiäthylensorbitanmonopalmitat | 30,0 % |
| p-Hydroxybenzoesäuremethylester | 0,065% |
| p-Hydroxybenzoesäurepropylester | 0,035% |
| Propylenglykol | 3,0 % |
| Wasser | ad 100,0 % |

Beispiel C: Lotion

| | |
|---|---|
| 17α,21-Tetrahydropyranyliden-2',2'-dioxy--4-pregnen-11β-ol-3,20-dion | 0,2 % |
| Paraffinöl dickflüssig | 10,0 % |
| Äthanol | 2,0 % |
| Glycerin | 1,0 % |
| Propylenglykol | 2,0 % |
| Sorbinsäure | 0,15 % |
| Fettalkoholpolyglykoläther | 2,0 % |
| Gemisch aus Cetylstearylalkohol und cetylstearylschwefelsaurem Natrium und nichtionogenem Emulgator | 0,5 % |
| Maiglöckchenparfümöl | 0,01 % |
| Wasser | ad 100,0 % |

Beispiel D: Salbe

| | |
|---|---|
| 17α,21-Tetrahydropyranyliden-2',2'-dioxy--4-pregnen-11β-ol-3,20-dion | 0,1 % |
| Gentamycinsulfat (bezogen auf freie Gentamycinbase) | 0,1 % |
| Cetylalkohol | 2,4 % |
| Wollfett wasserfrei | 1,0 % |
| Paraffin dickflüssig | 15,0 % |
| Vaseline® weiss | ad 100,0 % |

**Patentansprüche für die Vertragsstaaten:**
BE, CH, DE, FR, GB, IT, NL, SE

1. Hydrocortison-orthoester der allgemeinen Formel I

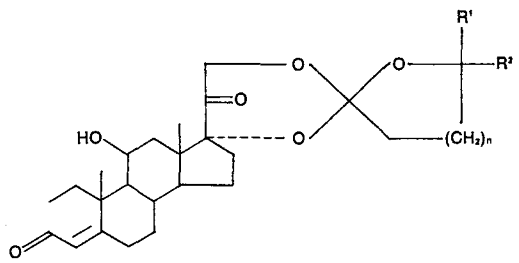

I

worin $R^1$ und $R^2$ jeweils H oder $CH_3$ und n 1 oder 2 bedeuten.

2. a) 17α,21-Tetrahydrofuranyliden-2',2'-dioxy--4-pregnen-11β-ol-3,20-dion;
b) 5'-Methyl-17α,21-tetrahydrofuranyliden--2',2'-dioxy-4-pregnen-11β-ol-3,20-dion;
c) 5',5'-Dimethyl-17α,21-tetrahydrofuranyliden-2',2'-dioxy-4-pregnen-11β-ol-3,20-dion;
d) 17α,21-Tetrahydropyranyliden-2',2'-dioxy--4-pregnen-11β-ol-3,20-dion.

3. Verfahren zur Herstellung von Hydrocortison-orthoestern der allgemeinen Formel I

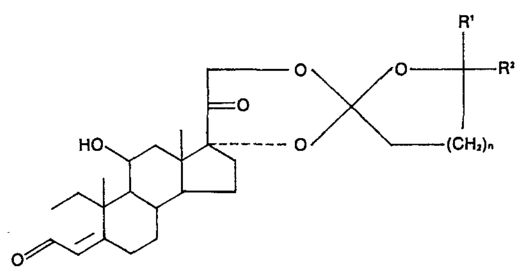

I

worin $R^1$ und $R^2$ jeweils H oder $CH_3$ und
n 1 oder 2
bedeuten,
dadurch gekennzeichnet, dass man Hydrocortison mit einem Lacton der allgemeinen Formel II

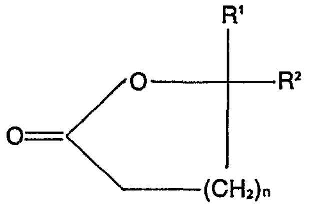

II

worin
$R^1$, $R^2$ und n die oben angegebene Bedeutung haben,
oder einem seiner reaktionsfähigen Derivate umsetzt.

4. Verfahren zur Herstellung pharmazeutischer Zubereitungen, dadurch gekennzeichnet, dass man eine Verbindung der Formel I zusammen mit mindestens einem festen, flüssigen oder halbflüssigen Träger- oder Hilfsstoff und gegebenenfalls in Kombination mit einem oder mehreren weiteren Wirkstoffen in eine geeignete Dosierungsform bringt.

5. Pharmazeutische Zubereitung, gekennzeichnet durch einen Gehalt an mindestens einer Verbindung der allgemeinen Formel I.

**Patentanspruch für den Vertragsstaat:** AT

Verfahren zur Herstellung von Hydrocortisonorthoestern der allgemeinen Formel I

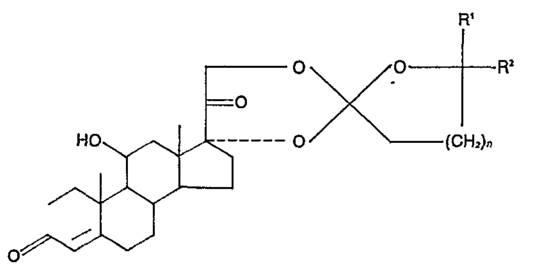

I

worin $R^1$ und $R^2$ jeweils H oder $CH_3$ und
n 1 oder 2
bedeuten,
dadurch gekennzeichnet, dass man Hydrocortison mit einem Lacton der allgemeinen Formel II

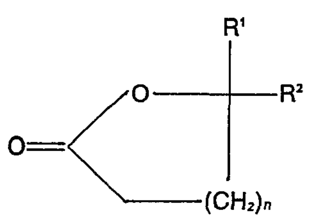

II

worin
$R^1$, $R^2$ und n die oben angegebene Bedeutung haben,
oder einem seiner reaktionsfähigen Derivate umsetzt.

**Claims for the Contracting States:**
BE, CH, DE, FR, GB, IT, NL, SE

1. Hydrocortisone orthoesters of the general formula I

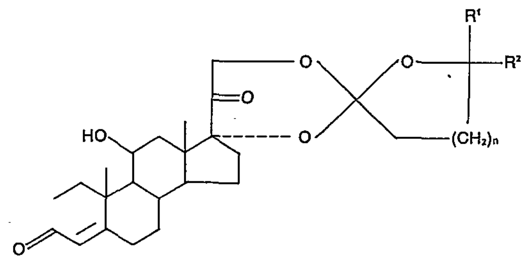

I

in which $R^1$ and $R^2$ in each case are H or $CH_3$ and n is 1 or 2.

2. a) 17α,21-Tetrahydrofuranylidene-2',2'-dioxy-4-pregnen-11β-ol-3,20-dione;
b) 5'-Methyl-17α,21-tetrahydrofuranylidene-2',2'-dioxy-4-pregnen-11β-ol-3,20-dione;
c) 5',5'-Dimethyl-17α,21-tetrahydrofuranylidene-2',2'-dioxy-4-pregnen-11β-ol-3,20-dione;
d) 17α,21-Tetrahydropyranylidene-2',2'-dioxy-4-pregnen-11β-ol-3,20-dione.

3. Process for the preparation of hydrocortisone orthoesters of the general formula I

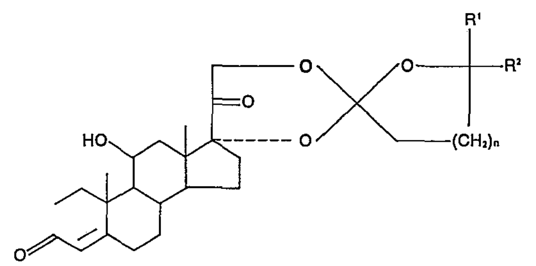

I

in which $R^1$ and $R^2$ in each case are H or $CH_3$ and n is 1 or 2, characterised in that hydrocortisone is reacted with a lactone of the general formula II

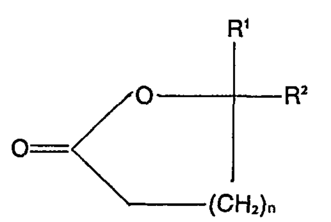

II

in which $R^1$, $R^2$ and n are as defined above, or with one of its reactive derivatives.

4. Process for the preparation of pharmaceutical formulations, characterised in that a compound of the formula I is brought into a suitable dosage form together with at least one solid, liquid or semi-liquid excipient or auxiliary and optionally in combination with one or more further active compounds.

5. Pharmaceutical formulation, characterised in that it contains at least one compound of the general formula I.

**Claim for the Contracting State:** AT

Process for the preparation of hydrocortisone orthoesters of the general formula I

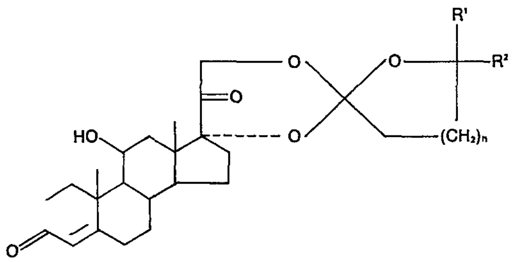

I

in which $R^1$ and $R^2$ in each case are H or $CH_3$ and n is 1 or 2, characterised in that hydrocortisone is reacted with a lactone of the general formula II

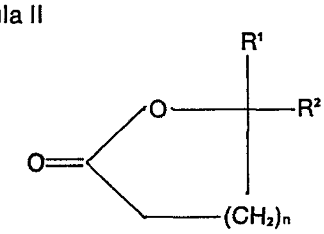

II

in which $R^1$, $R^2$ and n are as defined above, or with one of its reactive derivatives.

**Revendications pour les Etats contractants:** BE, CH, DE, FR, GB, IT, NL, SE

1. Orthoesters d'hydrocortisone de formule générale I

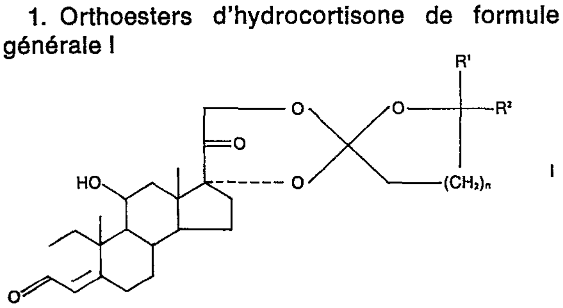

I

où $R^1$ et $R^2$ représentent chacun H ou $CH_3$ et n vaut 1 ou 2.

2. a) 17α,21-tétrahydrofuranylidèn-2',2'-dioxy-4-prégnèn-11β-ol-3,20-dione;
   b) 5'-méthyl-17α,21-tétrahydrofuranylidèn-2',2'-dioxy-4-prégnèn-11β-ol-3,20-dione;
   c) 5',5'-diméthyl-17α,21-tétrahydrofuranylidèn-2',2'-dioxy-4-prégnèn-11β-ol-3,20-dione;
   d) 17α,21-tétrahydropyranylidèn-2',2'-dioxy-4-prégnèn-11β-ol-3,20-dione.

3. Procédé de préparation d'orthoesters d'hydrocortisone de formule générale I

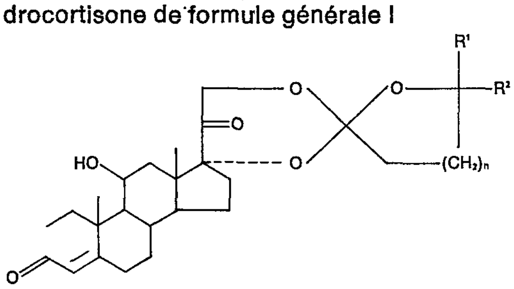

I

où $R^1$ et $R^2$ représentent chacun H ou $CH_3$ et où n vaut 1 ou 2, caractérisé en ce qu'on fait réagir de l'hydrocortisone avec une lactone de formule générale II

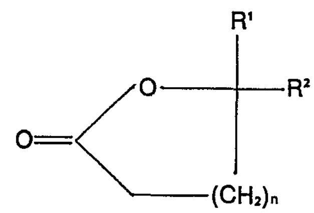

II

où $R^1$, $R^2$ et n ont la signification donnée ci-dessus, ou avec un de ses dérivés réactifs.

4. Procédé de fabrication de préparations pharmaceutiques, caractérisé en ce qu'on amène sous une forme posologique appropriée un composé de formule I avec au moins un support ou additif solide, liquide ou semiliquide éventuellement en combinaison avec une ou plusieurs autres substances actives.

5. Préparation pharmaceutique caractérisé en ce qu'elle contient au moins un composé de formule générale I.

**Revendication pour l'Etat contractant:** AT

Procédé de préparation d'orthoesters d'hydrocortisone de formule générale I

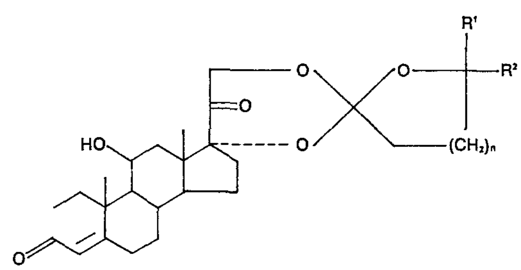

I

où $R^1$ et $R^2$ représentent chacun H ou $CH_3$ et où n vaut 1 ou 2, caractérisé en ce qu'on fait réagir de l'hydrocortisone avec une lactone de formule générale II

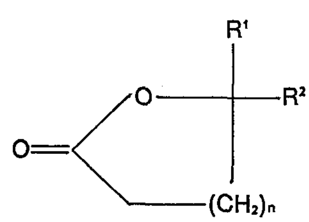

II

où $R^1$, $R^2$ et n ont la signification donnée ci-dessus, ou avec un de ses dérivés réactifs.